# EUROPEAN PATENT SPECIFICATION

(11) **EP 3 465 357 B1**
(45) Date of publication and mention of the grant of the patent: **22.05.2024**
(21) Application number: 17729420.4
(22) Date of filing: 31.05.2017
(51) Int. Cl.: G04G 21/02

(54) **WEARABLE DEVICE WITH AIR QUALITY SENSOR**
WEARABLE-VORRICHTUNG MIT LUFTQUALITÄTSSENSOR
DISPOSITIF POUVANT ÊTRE PORTÉ AYANT UN CAPTEUR DE QUALITÉ DE L'AIR

(30) Priority: 01.06.2016 US 201614170479
(43) Date of publication of application: 10.04.2019
(73) Proprietor: Withings, 92130 Issy les Moulineaux (FR)
(72) Inventor: BALTI, Haïkel, 94400 Vitry Sur Seine (FR); LEGER, Charlotte, 92120 Montrouge (FR); TECHER, Frédéric, 77120 Coulommiers (FR); BUARD, Nadine, 92190 Meudon (FR); BESNARD, Marc, 75006 Paris (FR)
(74) Representative: Withings IP
(86) International application number: PCT/EP2017/063162
(87) International publication number: WO 2017/207632

(56) References cited:
- WO-A1-2015/160830
- CN-U- 204 009 395
- CN-U- 204 086 834
- KR-B1- 101 598 908
- US-A1- 2015 174 517

## Description

### FIELD OF THE INVENTION

The present invention concerns a wearable device, for example a wrist watch or an activity tracker, with an embedded air quality sensor. Here, the term '*air quality sensor*' encompasses any sensor able to determine a concentration level of some chemical compound/species in the air which can have an impact on human health (otherwise called "pollutants"), among them for example NO₂, O₃, NOx, CO, SO₂, and others, without excluding chemical components which can have a long term impact on mankind's environment like CO₂, O₃, etc.

### BACKGROUND OF THE DISCLOSURE

Recently, some have suggested to include an air quality sensor into a device like a wrist watch. However, attempts to include an air quality sensor into a wrist watch remain rather unrealistic proposals and/or exhibit a rather bulky configuration. The documents WO2015/160830A1 and CN204009395U provide examples of wearable air quality control devices.

Furthermore, the attempts to embed an air quality into a wrist watch have not taken into account various everyday life environmental conditions to which the wrist watch (or `wearable device') is submitted. For example, a wrist watch is exposed to water when the user washes or rinses his/her hands. Sometimes the user sweats and the surface of the watch adjacent to the skin may become wet or even dirty over time.

Also, a wrist watch is usually exposed to mechanical stress like shocks and free falls, and therefore all the elements exposed to the outside must be rugged and solid.

Also, a wrist watch must remain in a size compatible with male and female proportions.

Also, many recently marketed multi-function watches or smart watches tend to have an insufficient energy autonomy, requiring frequent recharges or battery replacement.

Finally, it is desirable that the basis for air quality evaluation by the sensor should be substantially in real time, and thus the air sample subject to analysis shall be renewed frequently enough.

Therefore, there remains a need to provide a watch or a wearable device with an embedded air quality, robust and providing reliable measurements for the air quality, whatever the condition of use regarding the watch.

### SUMMARY OF THE DISCLOSURE

According to one aspect of the present invention, there is disclosed a device comprising a housing, in accordance with claim 1.

It is to be noted that the device is to be broadly construed. The device may be a wearable e.g. a watch, a wrist watch, an activity tracker, or other wrist-worn device, without excluding other types of personal wearable devices (glasses, necklace, ring, etc..).

Thanks to these dispositions, the air quality sensor is protected inside the housing of the wearable device against damages. Namely, the wearable device can withstand shocks or falls without negative impact on performance. The air quality sensor is protected inside the wearable device, whereas, at the same time, the sensor is able to sense various concentrations of chemical compounds/species present in the air outside the wearable device, thanks to the membrane being permeable to air and thus allowing the passage of gas to the cavity internal area.

Furthermore, the user can for example immerse briefly his wrist wearing the watch (or generally wearable device) in water without damaging the sensor since the membrane forms a barrier against ingress of water.

Moreover, the arrangement of the sensor in the cavity of the housing enables to perform a reliable measurement of pollutants.

The wearable device can be used to detect pollutants in the general environment of the user, and/or to detect some chemical species present in the breath of the user when the user exhales or blows toward the wearable device.

In various embodiments of the invention, the following described features may be used alone or in combinations other than the combinations explicitly described.

According to one option, the internal area of the cavity is in fluid communication with the ambient air exclusively through the membrane, the cavity being elsewhere isolated from the inside of the housing. Therefore, the passage of gas to the internal area of the cavity is exclusively through the membrane. Thereby, possible polluting gases that may come from the inside of the watch itself (e.g. from electronic component or battery or coating material) can be prevented.

In accordance with the present invention, the membrane is not exposed directly to the outside environment, the main casing offers mechanical protection together with fluid communication; air passage is enabled across the through bores toward the membrane, and further across the membrane toward the air quality sensor. Air renewal is sufficient for real-time analysis.

According to one option, the air quality sensor is able to sense and determine the concentration of NO₂ and O₃. Such a dedicated focused sensor may exhibit a small size and a very low electrical consumption.

According to one option, the air quality sensor is able to sense and determine the concentration of CO and/or NO₂ and/or SO₂. Thereby, the concentration of most common air pollutants can be determined, and according to a further option reported wirelessly to a mobile device of a user.

According to one option, the air quality sensor includes an amperometric sensor with a power consumption below 5 micro-amps. Thereby, the lifetime of the battery can be substantially improved if compared with some other wearable devices including gas sensor(s).

According to one option, the air quality sensor includes an amperometric sensor with a volume less than 0.2cm³. Thereby, such sensor can be integrated in a standard watch volume, even in a standard women watch volume.

According to one option, when the wearable device may be a wrist-worn device, the cavity wall is delimited by a dial plate and by the air quality sensor, the dial plate defining a top portion and a side portion of the cavity wall, a bottom portion of the cavity wall being formed by the air quality sensor, the interface between the sensor and the dial plate being locally sealed, the cavity wall comprising on the side portion an opening closed off by the membrane. Thereby, the dial plate provides an integrated solution to lodge the air quality sensor and to provide an air cavity. The bill of material and manufacturing process are thereby optimized.

According to one option, the interface between the sensor and the dial plate is sealed using at least one gasket.

An elastomeric gasket in a groove is a reliable solution to provide fluid tightness.

According to one option, there is provided a shoulder formed in the dial plate around the opening from a surface of the dial plate opposed to the cavity wall, the membrane being tightly received against the shoulder. It is therefore easy to glue, solder by ultrasonic vibrations, or otherwise affix the membrane on the shoulder so that it closes tightly the cavity and the border of the opening.

According to one option, there is provided an inner space located between the membrane and the perforated area of the main casing, the inner space being insulated from the inside of the housing using a gasket inserted between the housing and the dial plate close to the opening.

This provides tolerance for possible manufacturing play entailed by deviations of dimensions in an industrial process.

According to one option, the device may comprise a display arrangement which may comprise a plurality of physical stick-like hands forming analog indicators and at least a digital display area. This kind of mixed solution may enhance comfort and readability from the user standpoint.

The term "display arrangement" should be here construed broadly. According to one embodiment, the display arrangement may comprise analog type indicators with physical stick-like hands forming analog time display indicators and/or an auxiliary hand to display a current amount of physical activity performed by the user wearing the watch. According to another embodiment, the display arrangement may comprise digitalized pixel area, able to display a large variety of items, for example (but not necessarily) virtual hands for time indication.

According to still another embodiment the display arrangement may comprise both a digitalized pixel area, and physical stick-like hands forming analog indicators (for time and/or other data).

According to one option, each through bore has may have a dimension comprised in the range 0.05mm to 1.5mm; an example range for the bore diameter is [0.5mm, 1.5mm]. Although the through bores can have any diameter, this example range avoids a water meniscus which could obstruct the bore after exposition to water.

According to one option, the device comprises an electronic board. The electronic board may comprise a hole, the size of the hole being adapted for receiving the air quality sensor through said hole, the sensor emerging from both sides of the electronic board.

We obtain therefrom an integration of the air sensor, a compact piling up of components, enabling a thin package and giving a low thickness of the watch.

The display arrangement may further comprise a multi-color indicator LED, whose color is controlled by the electronic board according to the sensed level of air pollutants in the sampled air; whereby the user can have a very simple feedback of the current air quality around him/her.

The electronic board may be configured to generate one or more notifications for a user in response to a level of one or more air pollutants sensed by the air quality sensor.

The watch may further comprise a vibrator, controlled by the electronic board which is configured to activate the vibrator in case the sensed level of pollutants exceeds a predefined threshold; whereby the user can be warned at any time, even though he/she does not look at his/her watch.

According to one option, the air quality sensor may be able to determine the concentration of NO, and, whenever the user exhales towards the wearable device, the wearable device may be used to detect asthma, emphysema or similar lung disease from the breath of the user.

The device may further comprise a photo plethysmographic (PPG) sensor configured to capture a PPG data signal indicative of a user's heart rate; wherein the electronic board is further configured to: associate the PPG data signal over time with corresponding data of levels of one or more air pollutants sensed by the air quality sensor; and store or transmit the associated PPG data signal and the corresponding data of levels of one or more air pollutants sensed by the air quality sensor.

The device may further comprise an electrode configured to capture an electrocardiographic (ECG) data signal indicative of the user's heart rate; wherein the electrode is powered on in order to capture the ECG data signal indicative of the user's heart rate based on a determination that the PPG data signal indicative of the user's heart rate includes at least one cardiac arrhythmia; wherein the electronic board is further configured to: associate the ECG data signal over time with the corresponding data of levels of one or more air pollutants sensed by the air quality sensor; and store or transmit the associated ECG data signal and the corresponding data levels of one or more air pollutants sensed by the air quality sensor.

According to another aspect of the present invention, there is disclosed a method comprising: receiving a PPG data signal indicative of a user's heart rate; receiving data of levels of one or more air pollutants; associating the PPG data signal over time with corresponding data of the levels of one or more air pollutants; and storing or transmitting the associated PPG data signal and the corresponding data of the levels of one or more air pollutants.

The method may further comprise: determining that the PPG data signal indicative of the user's heart rate includes at least one cardiac arrhythmia; in response to said determination, obtaining an ECG data signal indicative of the user's heart rate; associating the ECG data signal over time with the corresponding data of the levels of one or more air pollutants; and storing or transmitting the associated ECG data signal and the corresponding data levels of the one or more air pollutants.

According to another aspect of the present invention there is provided a computer program that when run on a computer enables any of the above described methods to be performed.

### BRIEF DESCRIPTION OF THE DRAWINGS

Other features of the invention appear from the following detailed description of one of its embodiments, given by way of non-limiting example, and with reference to the accompanying drawings, in which:
- Figure 1 shows an elevation cross section of a wristwatch according to the present disclosure,
- Figure 2 shows an exploded view of the wristwatch of Figure 1,
- Figure 3a and 3b show perspective views of the dial plate, from the bottom site and respectively from two different viewpoints,
- Figure 4 shows an enlarged sectional view of the membrane area,
- Figure 5 shows an enlarged view of the air quality sensor,
- Figure 6 shows another perspective sectional view of the wristwatch of Figure 1,
- Figure 7 illustrates a use case with wireless upload of data from the wristwatch of Figure 1 to a smartphone,

### DETAILLED DESCRIPTION OF THE DISCLOSURE

In the figures, the same references denote identical or similar elements.

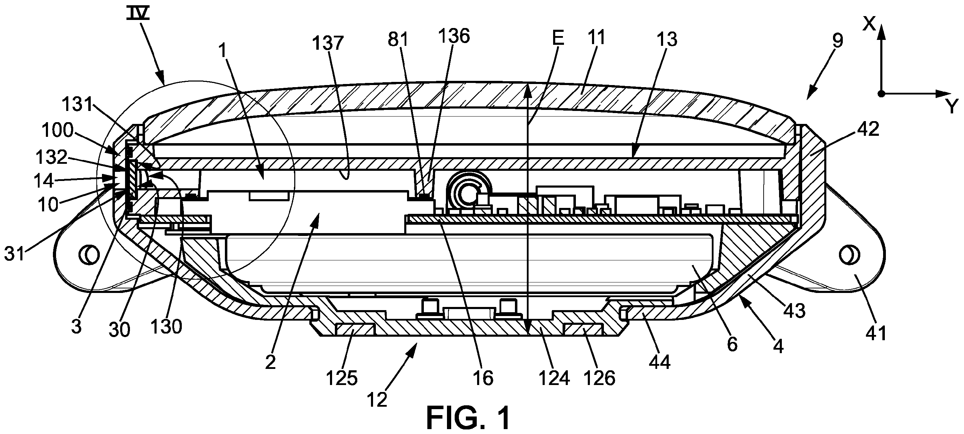

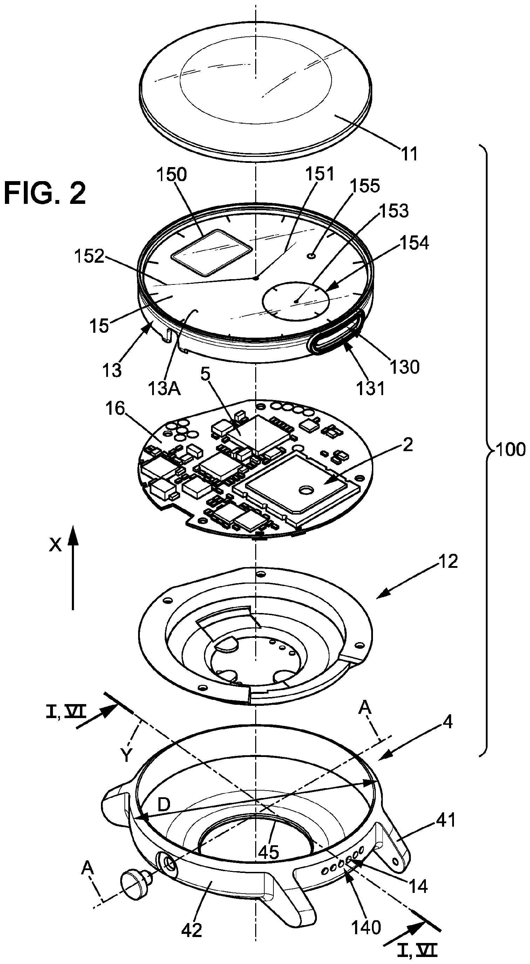

As illustrated on Figures 1 and 2, one exemplary embodiment of the present disclosure relates to a wrist watch **9**. The wrist watch in question may exhibit a standard size suitable for both men and women. An example of such a device is a watch name "Activité^{™}" from the present applicant "Withings^{™}". The body of the watch may have an external diameter **D** of 42mm or less; the thickness **E** of the body is 14mm or less. The thickness **E** may be 12,5 mm or 12 mm.

From the body, there may be provided projections **41** for attachment to a wristband 19 as known *per se*, thus not described in detail here.

The wrist watch **9** illustrated here is a *digital watch*, namely all the indications given on the front face of the watch are given by a high definition dot matrix display, of LED or OLED technology, which is referred to herein as display arrangement **15.**

Virtually, almost any object can be displayed using this type of display, pictograms, numbers, digitalized areas **150.** Also, virtual hands **151,152** (for hours and minutes display) can be displayed using such high definition display; there can be provided an auxiliary indicator 153 as well.

Alternately, the present invention can be applied to an *analog type watch*, that-is-to-say a watch where the time is indicated through a dial and solid stick-like hands (one for hours, one for minutes, and optionally one for seconds) driven by stepper motors and gear trains as known per se.

Alternately, the present invention can be used in a hybrid watch, having both solid stick-like hands and digitalized display area.

In the embodiment illustrated, the body of the watch is substantially round; but in other embodiments, the body of the watch can be substantially square or rectangle.

As illustrated, for example in in figure 2, the exemplified watch may comprise, from bottom to top:
- a main casing **4**,
- a back cup **12**,
- a battery **6,**
- a printed circuit board **16** and a sensory device **2,**
- a dial plate **13**, otherwise called "dial support",
- a top cover **11**, which is transparent.

The vertical direction denoted **X** is defined as the direction transverse to the plane of the watch dial. The thickness of the watch is defined along this vertical direction **X.**

Further, there is also defined a longitudinal direction denoted **Y**, perpendicular to X and which corresponds to the main direction of extension of the wristband **19** from the watch. Further, a third direction, perpendicular to the other ones, is named "transversal" and denoted **A.** The plane A,Y is referred to as a watch plane.

Further, a **housing 100** of the wrist watch 9 may include the main casing **4**, the back cup **12** and the top cover **11;** the housing can also be called "enclosure" since it encloses and protects all the internal components and elements contained in the watch.

As illustrated on figures 1 and 2, the main casing **4** may comprise a cylindrical portion **42** having a vertical axis; the cylindrical portion **42** is prolonged downwards by a tapered frustoconical portion denoted **43**; the tapered portion is further prolonged downward by a flat ring portion **44.** The flat ring portion **44** is shaped as a disc with X axis, and comprises a central opening **45.**

The main casing **4** may be manufactured in stainless steel; alternatively light alloy or high-performance plastics like ABS, or other robust material, can also be considered.

The back cup **12** may be formed like a cup with a bottom section inserted in the above mentioned central opening of the main casing. The bottom section may be prolonged upwards by a tapered portion adjacent internally to the tapered frustoconical portion of the main casing.

The back cup **12** may be manufactured from a plastic rigid material, for example ABS or polycarbonate.

The above-mentioned sensory device **2** may be an **air quality sensor 2** which is enclosed in the housing.

The air quality sensor **2** may be used to determine the rate of pollutants in the air prevailing at the vicinity of the watch. It should be noted that the samples collected at the watch are not substantially different from the air that the user/wearer of the watch is breathing.

More precisely, the air quality sensor 2 is enclosed in a cavity **1** provided in the housing 100.

The **cavity 1** is delimited by a **cavity wall** and by a membrane **3,** which is porous. The cavity **1** is in fluid communication with the ambient air through the membrane 3. That is, the membrane 3 allows a passage of gas to the cavity internal area. This will be described in detail later.

In use, the air enters the housing 100 of the watch and passes across the membrane **3.** The pollution rate is measured by the sensor **2** in the internal area of the cavity **1.**

According to one first possibility, the air quality sensor **2** can be a sensor mainly reactive to NO₂ (Nitrogen Dioxide). According to another possibility, the air quality sensor **2** can be a sensor mainly reactive to NO (Nitrogen Oxide).

According to another possibility, the air quality sensor 2 can be a sensor mainly reactive to SO2 (Sulfur Dioxide). According to another possibility, the air quality sensor 2 can be a sensor mainly reactive to CO (Carbon Monoxide).

According to another possibility, the air quality sensor 2 can be a sensor mainly reactive to H₂S (Hydrogen Sulfide).

According to other possibilities, the air quality sensor 2 can be a sensor mainly reactive to CO₂ (Carbon Dioxide) or a sensor mainly reactive to ozone (O₃) or a sensor mainly reactive to chlorine or a sensor mainly reactive to benzene, or a sensor mainly reactive to methane.

According to another possibility, the air quality sensor **2** can be a sensor reactive to two or more of the above mentioned chemical compounds and/or chemical elements.

According to one embodiment, the air quality sensor **2** may be reactive both to NO₂ and O₃, with a higher gas selectivity than known general-purpose pollutants sensors, like for example the so-called VOC (volatile organic compounds) sensors.

The physics of the sensor(s) relies on electrochemical reactive process, with a chemical layer combined with a transducer, for example an amperometric sensor.

A working electrode (or catalyst) promotes a chemical reaction when in contact with the gas, which takes place with regard to a second electrode (common = counter electrode).

For example, respective chemical reaction for CO and NO₂ are:

CO + H₂O <=> CO₂ + 2e- + 2H+ (electrons are released in the working electrode)

NO₂ + 2e- + 2H+ <=> NO + H₂O (electrons are captured in the working electrode)

Chemical interaction velocity follows the Arrhenius law with a certain activation energy E which depends on many parameters: Kc (speed)=Aexp(-E/RT)

The kinetics of a given chemical reaction makes a corresponding sensor respond immediately to a certain gas and very slowly to another one.

A bias reference voltage may be set to the working electrode which results in a current flowing from/to the working to the ground electrode, the current depending on the kinetics of the considered chemical reaction.

Electrolyte may be provided between the two electrodes to transport the H+ ion from one electrode to the other electrode.

According to one exemplary choice, one can select product reference 3SP_NO₂_20 from SPEC Sensors, LLC, of Newark, CA. Due to its fuel-cell like technology, the air quality sensor 2 may exhibit a very low electrical consumption, for example an average consumption when powered of less than 5 microAmps, or less than 2 microAmps.

If we consider additionally the polarisation current from the main PCB, the total amount of current used for the sensor is may be less than 10 microAmps. Therefore, with a conventional lithium battery **6** for a watch, for example of the type CR2025 or CR2032 ("button cell"), the lifetime of the battery may be more than one year, for example around 18 months or even two years.

It should be noted that in one embodiment, even though the sensor may be permanently supplied, the sampling of sensor signal may be made from time to time to lower further the electrical consumption.

It is important to note that more than one sensor can could be arranged in the air cavity, for examples two sensors or more.

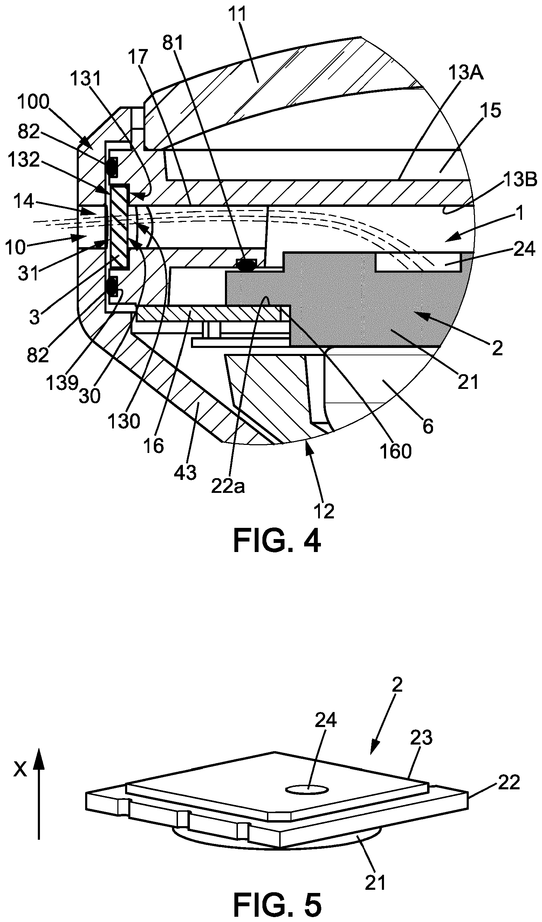

As shown on Figure 5, the exemplified air quality sensor **2** may be composed of three sections, one above the other along the vertical direction. The lower section denoted **21** is in form of a disc and has a circular periphery. The lower section houses an electrochemical cell comprising a reactant like an electrolyte or a gel.

The lower section is followed upwards by an intermediate section **22**, having a rectangular shape. The intermediate section **22** is a ring shaped thin PCB for electrical connection to the main printed circuit board 16 (soldering face-to-face).

A top portion **23** has also rectangular shape, but with smaller dimensions in the shown example. The top portion **23** forms a cover and comprises a recess **24** opened in the surface of the top portion to let the air enter the sensitive part below.

The largest section of the air quality sensor 2 may exhibit dimensions in the plane **A,Y** of the watch, comprised between roughly (5mm × 5mm) and (15 mm × 15mm), for example around 10mm × 10mm.

A mechatronic integration solution will be explained further.

However, it should be noted that the geometric configuration of the air quality sensor 2 can be slightly different or completely different.

In the shown example, the cavity **1** is delimited by the dial plate **13** and by the air quality sensor **2.** The dial plate **13** defines a top portion **137** and a side portion **136** of the cavity wall. A bottom portion of the cavity wall is formed by the air quality sensor 2 itself.

The dial plate 13 is for example formed in polycarbonate. Although, various other materials can be considered for the dial plate.

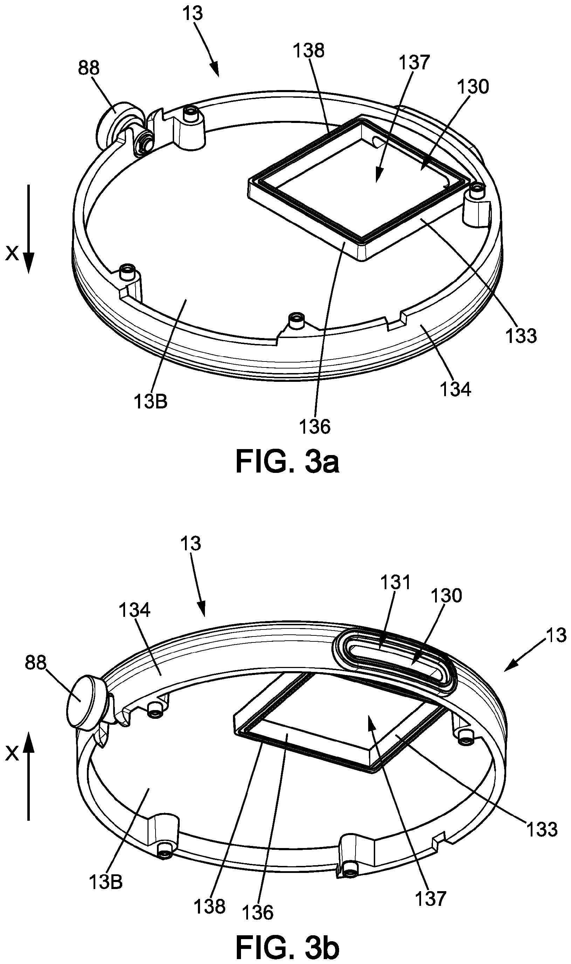

The dial plate **13** is illustrated on figures 3a and 3b in the case of wrist watch housing with a circular shape. The dial plate may be a disc with a peripheral rim **134.** The disc may have a diameter slightly inferior to the internal diameter of the cylindrical portion 42 of the casing 4.

The disc extends in the plane A,Y of the watch with a top side **13A**, in which the display arrangement **15** can be arranged and a bottom side **13B** which faces the printed circuit board **16.**

A border **133** may extend from the bottom side **13B** of the disc downwards in the vertical direction on a length inferior to the height of the peripheral edge from the bottom side of the disc. The border **133** delimits the cavity. The border **133,136** defines in this embodiment a rectangular shape.

The cavity has in this embodiment a square shape footprint with side size between 0.5cm and 1.5cm.

Alternatively the cavity could have a circular section.

The cavity **1** is closed by the sensor **2** at the bottom side of the cavity **1.** For example, the sensor **2** surface **22a** may be in contact with the lower end of the border **133**, and all along the periphery of the border 133. It is possible to provide a glue or silicone bead (or other seal) to provide a tight junction.

Referring to Figures 1 and 4, the top section **23** may protrude slightly in the cavity 1. The extremity of the border **133** opposed to the disc comprises a groove **138** along the extremity of the border **133.**

A gasket **81** is for example inserted along this junction area between the sensor and the border **133.** The gasket **81** has for example a rectangular loop shape matching with the shape of the border of the cavity. The gasket **81** has for example a round cross-section, configured to be inserted in the above mentioned groove **138.**

The material of the gasket is chosen to be neutral versus the detection of chemical compounds; some example materials are Viton^{™}, EPDM (ethylene propylene diene monomers), or silicon.

In this embodiment the thickness of the air sensor is for exmaple between 2mm and 4mm.

One side of the cavity wall formed by the border **133** may be formed proximate to the peripheral rim **134** of the disc such that there is a material continuity and the border 133 and the peripheral rim 134 are locally the same.

A **tunnel 17** may be open from the internal area of the cavity 1 through the cavity wall locally and then in continuity through the peripheral rim **134**, ending in an **opening 130** at the outward surface of the peripheral rim **134.** The tunnel **17** connects the internal area of the cavity with the opening **130.**

The membrane **3** closes off the cavity wall opening. The membrane **3** is permeable to air and not permeable to water. The membrane **3** comprises a porous material.

The membrane **3** may be formed as a thin wall manufactured in synthetic material having a microporous structure. Across the membrane, diffusion is governed by Fick's law.

Various materials are available to achieve water protection while allowing air passage. According to one example, the membrane is made of polytetrafluoroethylene (also called Teflon^{™}) marketed under Gore-Tex^{™} membranes. According to another example, the membrane can be made of non-woven high-density polyethylene fibers. Nanopores having a size comprised between 1 µm and 5 µm let the way for air molecules while retaining bubbles of water. The thickness of the membrane **3** may be, for example, between 50µm and 500µm.

The 'Schmerber' index, denoting water non-permeability performance, may be at least 5 000 ; at which value water cannot pass through the membrane for a pressure difference threshold up to 200 mbar. This pressure difference may be chosen to be 270 mbar.

Likewise, IP rating of the membrane **3** may be at least IP64, for example IP67 or IP68.

Air permeability may be at least 70 liter/hour/cm² at a 70 mbar of pressure difference.

An inner wall **30** of the membrane **3** is oriented toward the cavity internal area and an outer wall **31** is opposed to the inner wall. The internal area of the cavity is in fluid communication with the ambient air through the membrane **3.** That is, the membrane **3** allows a passage of gas to the cavity internal area.

A shoulder **131** is formed around the opening **130.** The shoulder **131** is formed at the peripheral rim **134** from the surface of the peripheral edge of the opening **130.** The membrane **3** is in this embodiment inserted in the shoulder **131** in a way wherein the membrane **3** is substantially flush with the surface of the peripheral rim **134.**

Alternatively the periphery of the inner wall **30** of the membrane can be welded with ultrasound to the surface of the peripheral rim **134** to close off the cavity.

The opening **130** has in this embodiment an oval shape extending along an axis parallel to the watch plane. So the membrane has a typical oval shape in this embodiment to be placed against the described shoulder 131.

Alternatively the opening can have a different shape, such as a rectangular shape.

A sealing gasket **82** may be additionally arranged on a peripheral groove **139** which encompasses the opening **130** and its shoulder **131.**

In the illustrated case of a digital watch, any type of object can be displayed as mentioned above.

In the analog type variant, the top side **13A** of the dial plate **13** supports in this embodiment the physical pointers 151,152 of the watch for example.

The transparent cover **11** covers the pointers in a way wherein the pointers are visible through the transparent cover **11.**

The peripheral rim **134** on the top side of the dial plate **13** comprises a shoulder, as illustrated on figures 3b and 4, adapted to insert the peripheral edge of the transparent cover **11** in a watertight way.

The dial plate **13** is enclosed in the housing such as that the disc is parallel to the plane of the watch. In this way wherein the peripheral rim **134** is parallel to the lateral part of the main casing **10** formed by the cylindrical part. The opening **130** faces the lateral part. The membrane **3** is thus parallel to the main casing locally.

The lateral part of the main casing **10** has a vertical extension comprised between 4mm to 6 mm. The thickness of the watch is roughly the height of the main casing **10** which is in the range of 8mm to 14mm.

The main casing 4 is for example obtained from stamping in one piece of stainless steel. The lateral part of the main casing **10** comprises a perforated area **140**, wherein through bores **14** are open to connect an internal area of the housing **100** to outside.

The through bores 14 may be aligned in the perforated area **140**, facing the opening **130**. There are for example six bores. Each bore 14 may have a diameter comprised between 0,05 mm and 1.5 mm, for example about 1mm. The through bores **14** are separated by about 1.5 mm.

The internal area of the cavity **1** communicates with outside through the through bores **14** and through the membrane 3. The air enters the housing **100** through the through bores 14 and then enters the cavity **1** through the membrane **3.**

In an example embodiment, the diameter of the through bore 14 is proportionately sized to the cavity 1, to avoid a meniscus effect. The oval opening **130** may have an extension in a range of 1cm to 1.5cm, for example of around 1.2cm. Alternatively the bores could be replaced by a slit.

An inner space **132**, illustrated on figure 4, is in this embodiment located between the membrane 3 and the perforated area **140.**

This inner space **132** is isolated from the rest of the internal components by the above mentioned gasket **82** and possible polluting gases that may come from the inside of the watch itself (e.g. from electronic component or battery or coating material) is thereby prevented

The housing 100 may enclose several other items as an electronic board (printed circuit board, PCB) 16 with a controller **5**, an oscillator, a battery **6** either conventional or rechargeable, etc.

The battery of the watch may be below the PCB inside the cavity in the bottom part of the housing. Gas is for example possibly emitted by the PCB or by the battery and could disturb the measurement by the sensor 2.

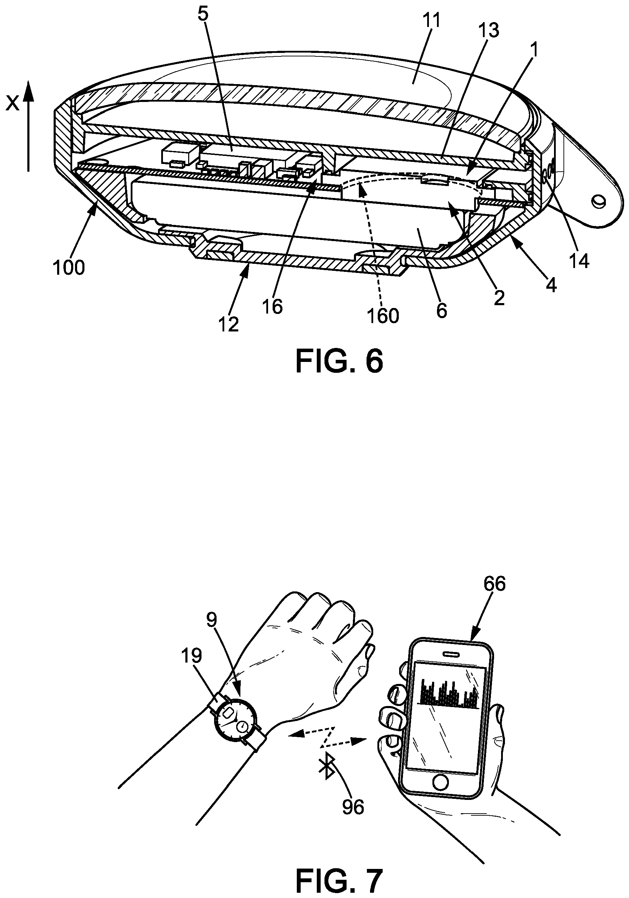

As illustrated on figure 6, the PCB may be positioned between the battery and the cavity, parallel to the plane of the watch. The PCB may comprise a hole 160 in which is inserted the sensor 3. In this configuration, the periphery of the bottom side of the intermediate portion is in contact with the PCB surface. The sensor extends through the PCB in a way wherein the bottom face of the air quality sensor is vertically below the PCB 16.

In this configuration the thickness of the watch housing 100 is minimized with the integration of the air quality sensor through the PCB.

The sensor 2 is connected to a control unit **5** integrated to the PCB 16 which collects and treats air quality data. A diode **155** is for example mounted on the dial plate top face and connected to the control unit. The diode is used to provide for example a light color signal representative of the air quality measured.

As an alternative or complementarily, the watch may be wirelessly connectable **96** to another device like a smartphone **66**, or other devices, and the data could be displayed on the screen of the connected device as illustrated in figure 7.

Instead of, or in adiditon to a single LED 155, a bargraph or other display can be used. Also, the actual sensed values of pollutants can be displayed on the digitalized area **150.**

On the smartphone **66**, there may be provided histograms of pollutant rate along the present day, along the past three days, along the past week etc.

Additionally, there may be displayed for each pollutant compound, a threshold line showing the predefined alert level, with different colors on histograms whenever the pollutant rate become higher than the threshold(s).

Optionally, there may be provided one or more user actuator **88** such as a button; various purposes can be allocated to actuation of such actuator, as known per se, like time setting, menu browsing, items selection, or the like. User interface can also rely on tap actuations using an embedded accelerometer.

In the analog type version, stepper motors and gear trains are arranged for example on the side of the watch plane opposed to the side where the air quality sensor is placed.

Optionally, the watch is equipped with a photo plethysmograpic (PPG) sensor **125** used to sense the heart rate of the user of the watch. The PPG sensor is arranged on the bottom face of the watch.

Optionally, the watch is equipped with an electrode **124** in contact with the user's skin to form a sensor used for electrocardiographic (ECG) analysis. The electrode for ECG sensing is also arranged on the bottom face of the watch.

Optionally, the watch is equipped with a temperature sensor **126**, configurable to measure the skin temperature on the wrist of the user. The skin temperature sensor is also arranged on the bottom face of the watch. There may be provided additionally an ambient temperature sensor.

Optionally, the watch is equipped with a vibrator, which can be used to notify the user with a message or an alert.

Regarding the predefined thresholds to trigger an alert, the following values can be taken into account. These are examples.

For NO₂, a first threshold value is set at 200µg/m³.

For NO₂, a second threshold value is set at 400µg/m³.

Each of the first and second NO₂ thresholds can be considered in conjunction with a minimum exposure time, for example an exposure duration or a certain number of times when the threshold is exceeded.

For O₃, a first threshold value is set at 240µg/m³.

For O₃, a second threshold value is set at 300µg/m³.

For O₃, a third threshold value is set at 360µg/m³.

Each of the first, second and third O₃ thresholds (abovementioned) can be considered in conjunction with a minimum exposure time, for example an exposure duration or a certain number of times when the threshold is exceeded.

Once a particular threshold is exceeded with its associated time condition(s), a local message can be given by vibrations, and/or by a particular color on the indicative LED. Also, one or more messages can be sent to the connected smartphone **66.**

In an example embodiment the device **9** may be configured to record and store data to a local storage memory. The data may be recorded in real time from the respective sensors of the device 9 (e.g. echocardiogram data signal from ECG electrode **124**, PPG data signal from PPG sensor **125**, and concentration levels of one or more air pollutants from the air quality sensor 2, as described above). The PCB electronic board 16 may be configured to monitor the recorded data. Abnormalities in a user's heart rate variability (HRV) could be an indication of a cardiac arrhythmia and can be detected by monitoring the PPG and/or ECG data signals. Although the following describes monitoring for and detecting cardiac arrhythmia, it should be understood that other changes or patterns in the user's heart rate can be detected by monitoring the PPG and/or ECG data signals.

In embodiments, the PPG sensor may initially collect PPG data signals on its own and without the ECG sensor in operation (or vice versa). If a cardiac arrhythmia is detected from the PPG data signal, the ECG signal may be used to validate that the user is actually suffering from a cardiac arrhythmia. For example, the ECG sensor electrode may be automatically powered on and monitor the user's heart rate on the condition that a cardiac arrhythmia has been detected from the recorded PPG data signal, for example over the course of a predefined number of heart beat cycles or over a predefined period of time e.g. the last one minute. This may allow the device **9** to conserve power as it avoids having both the ECG sensor and the PPG sensor set to a continuous mode of operation. The ECG sensor electrode may be manually powered off by a user, or automatically powered off if the electronic board 16 does not detect a cardiac arrhythmia from the ECG data signal after a predefined number of heart beat cycles or over a predefined period of time e.g. at least one minute.

As mentioned above, the recorded data may be stored on a device **9** local storage memory. Additionally or alternatively, the recorded data may be stored at one or more remote storage units. The device 9 may be configured to upload or transmit data it collects to the one or more remote storage units. This transmission of data may be performed in real time as the data is being collected from the user. Additionally or alternatively, data that has already been recorded, for example to the local storage memory of device 9, may be transmitted to one or remote storage units or other devices. The transmission of data may be made by wireless communication protocol (e.g. Wi-Fi network, LTE network, Bluetooth, NFC or the like); a wired connection (e.g. LAN, Ethernet, USB, micro USB, etc.), or transferred via a removable memory card or the like (e.g. Secure-Disk micro card, etc.). Although embodiments refer to transmitting and uploading data from the device **9**, it should be understood that in alternative embodiments the data may be received or downloaded by another device and/or the remote storage unit(s).

The recorded data can further include timestamp data so that data points in the data signals recorded from the PPG sensor and/or ECG sensor, and data points in the concentration levels of the one or more air pollutants can be analyzed over time. Therefore the user and/or another person can perform an analysis of the data as recorded over time. For example, the other person may be a clinician or healthcare professional who is trained to analyze such recorded data. Additionally or alternatively, a computing device may be configured to perform an analysis of the data as recorded over time. The user, other person and/or computing device may be able to identify one or more correlation points or trends in the recorded data. For example there may be a correlation between instances of cardiac arrhythmia and peak concentrations of one or more air pollutants. This could prompt the user to take action to avoid further exposure to the environment(s) where the peak levels of air pollutants was recorded. It should be noted that there may or may not be a causal link between a detected arrhythmia and the corresponding detected concentration level of the one or more air pollutants. Therefore any such correlations should be treated with some caution. The computing device for analyzing the recorded data may be the electronic board 16 of the device 9 or another device such as the smartphone 66. Additionally or alternatively, the other device could any suitable computing device.

The electronic board **16** or remote computing device may be further configured to automatically alert the user and/or other person to detected instances of cardiac arrhythmia and/or when a correlation between instances of cardiac arrhythmia and concentration levels of the one or more air pollutants. These alerts may be generated by the electronic board 16 and provided as a notification on the device **9** and/or the other device. For example the notification may be indicated by the device **9** on the display arrangement 15, by LED 155 and/or by a vibration etc. (as described above). Alternatively the alert may be generated by the other device and transmitted to the device **9** and/or indicated by the other device itself.

The device 9, and/or the other device such as the smartphone 66 may be configured to run a non-transitory computer program code that enables the recorded data to be visually rendered in one or more ways including but not limited to: list(s) of textual and/or numerical data, bar graph form, line graph form, chart form, or any other suitable type of visual representation. In some examples the display arrangement **15** of a wearable device **9** may be limited in size compared to a display screen of the other device. In such cases, the most relevant or critical data can be shown on the display arrangement 15 when it is relatively small in size, while more details and optionally larger format displays of the recorded data may be shown on a larger display associated with the other device. The computer program code and recorded data may also be configured so that the recorded data can be rendered audibly or via haptic feedback by the relevant devices.

As already stated, although the shown embodiment relates to a watch, the configuration of the membrane, the cavity and the air quality sensor can of course be applied to any kind of personal activity tracking device, or any other kind of device. For example, the device may be a fixed, portable, wearable and/or integrated device configured for use in a range of environments, including: indoors, outdoors, and/or in in a functional space e.g. around a home, public spaces and/or a workplace etc.

As already stated, the above configuration (for a watch or more generally for a wearable device) with the adequate amperometric gas sensor can also be used for breath analysis, when the user blows towards the wearable device. In some examples, the result can be determined faster when the user blows directly towards or into the bores **14.**

Breath analysis is a known technique to screen diseases. For example the presence of NO in the breath is a marker for asthma, emphysema or similar lung disease.

## Claims

1. A wearable device (9) comprising a housing (100),
the housing (100) having a cavity (1) with an internal area,
the cavity (1) containing an air quality sensor (2) in the internal area,
the cavity (1) being delimited by a cavity wall and by a membrane (3),
the membrane (3) having an inner wall (30) oriented toward the cavity internal area and an outer wall (31) opposed to the inner wall,
wherein the membrane (3) is permeable to ambient air and substantially not permeable to water, allowing a passage of gas to the cavity internal area,
the housing (100) is formed by a main casing (10), a top cover (11) and a back plate (12), and
the main casing (10) comprising at least one through bore (14) located in an opening area (140) for fluid communication between an internal area of the housing and outside, the outer wall of the membrane (31) facing the opening area (140).

2. The device according to claim 1 wherein the passage of gas to the cavity internal area is exclusively through the membrane (3), the cavity being elsewhere isolated from the inside of the housing (100).

3. The device according to any of claims 1 or 2, wherein the air quality sensor (2) is able to determine the concentration of NO₂ and/or CO and/or SOz and/or O₃.

4. The device according to any of claims 1 to 3, wherein the air quality sensor (2) includes an amperometric sensor with a power consumption below 5 micro-amps and/or with a volume less than 0,2 cm³.

5. The device according to any of claims 1 to 4, wherein the device is formed as a wrist-worn device.

6. The device according to any of claims 1 to 5, wherein the cavity wall is delimited by a dial plate (13) and by the air quality sensor (2), the dial plate (13) defining a top portion and a side portion of the cavity wall, a bottom portion of the cavity wall being formed by the air quality sensor, the interface between the air quality sensor (2) and the dial plate (13) being locally sealed, the cavity wall comprising on the side portion an opening (130) closed off by the membrane (3).

7. The device according to claim 6, wherein the interface between the sensor (2) and the dial plate (13) is sealed using at least one gasket (81).

8. The device according to any of claims 6 or 7, wherein a shoulder (131) is formed in the dial plate (13) around the opening (130) from a surface of the dial plate opposed to the cavity wall, the membrane (3) being tightly received against the shoulder.

9. The device according to claim 8, wherein an inner space (132) is located between the membrane (3) and the perforated area (140), the inner space (132) being insulated from the inside of the housing (100) using a gasket (82) inserted between the housing and the dial plate (13) close to the opening (130).

10. The device according to claim 1, wherein each through bore has a diameter comprised in the range 0.05 and 1.5mm.

11. The device according to any of claims 1 to 10, wherein the device comprises an electronic board (16), the electronic board (16) comprises a hole (160), the size of the hole being adapted for inserting the air quality sensor (2) through the hole, the air quality sensor emerging from both sides of the electronic board.

12. The device according to claim 11, further comprising a multi-color indicator LED (115), whose color is controllable by the electronic board (16) according to a level of one or more air pollutants sensed by the air quality sensor (2).

13. The device according to claim 11, further comprising a photo
plethysmographic (PPG) sensor (125) configured to capture a PPG data signal indicative of a user's heart rate; wherein
the electronic board (16) is further configured to:
associate the PPG data signal over time with corresponding data of levels of one or more air pollutants sensed by the air quality sensor; and
store or transmit the associated PPG data signal and the corresponding data of levels of one or more air pollutants sensed by the air quality sensor.

14. The device according to claim 13, further comprising an electrode (124) configured to capture an electrocardiographic (ECG) data signal indicative of the user's heart rate; wherein
the electrode is powered on in order to capture the ECG data signal indicative of the user's heart rate based on a determination that the PPG data signal indicative of the user's heart rate includes at least one cardiac arrhythmia; wherein
the electronic board (16) is further configured to:
associate the ECG data signal over time with the corresponding data of levels of one or more air pollutants sensed by the air quality sensor (2); and
store or transmit the associated ECG data signal and the corresponding data levels of one or more air pollutants sensed by the air quality sensor.

## Patentansprüche

1. Eine tragbare Vorrichtung (9) mit einem Gehäuse (100),
das Gehäuse (100) einen Hohlraum (1) mit einer Innenfläche aufweist,
der Hohlraum (1) enthält im Innenbereich einen Luftqualitätssensor (2),
wobei der Hohlraum (1) durch eine Hohlraumwand und eine Membran (3) begrenzt ist,
wobei die Membran (3) eine Innenwand (30), die zum Innenbereich des Hohlraums hin ausgerichtet ist, und eine Außenwand (31) aufweist, die der Innenwand gegenüberliegt, wobei
die Membran (3) für Umgebungsluft durchlässig und im Wesentlichen nicht wasserdurchlässig ist, so dass Gas in den Innenbereich des Hohlraums eindringen kann,
das Gehäuse (100) aus einem Hauptgehäuse (10), einer oberen Abdeckung (11) und einer Rückwand (12) besteht, und
das Hauptgehäuse (10) mindestens eine Durchgangsbohrung (14) aufweist, die sich in einem Öffnungsbereich (140) zur Fluidverbindung zwischen einem Innenbereich des Gehäuses und der Außenseite befindet, wobei die Außenwand der Membran (31) dem Öffnungsbereich (140) zugewandt ist.

2. Vorrichtung nach Anspruch 1, bei der der Durchgang des Gases zum Innenbereich des Hohlraums ausschließlich durch die Membran (3) erfolgt, wobei der Hohlraum ansonsten vom Inneren des Gehäuses (100) isoliert ist.

3. Vorrichtung nach einem der Ansprüche 1 oder 2, wobei der Luftqualitätssensor (2) in der Lage ist, die Konzentration von NO₂ und/oder CO und/oder SO₂ und/oder O₃ zu bestimmen.

4. Vorrichtung nach einem der Ansprüche 1 bis 3, wobei der Luftqualitätssensor (2) einen amperometrischen Sensor mit einer Leistungsaufnahme von weniger als 5 Mikroampere und/oder mit einem Volumen von weniger als 0,2 cm³ umfasst.

5. Vorrichtung nach einem der Ansprüche 1 bis 4, wobei die Vorrichtung als am Handgelenk zu tragende Vorrichtung ausgebildet ist.

6. Vorrichtung nach einem der Ansprüche 1 bis 5, **dadurch gekennzeichnet, dass** die Hohlraumwand durch eine Skalenplatte (13) und durch den Luftqualitätssensor (2) begrenzt ist, wobei die Skalenplatte (13) einen oberen Teil und einen seitlichen Teil der Hohlraumwand definiert, wobei ein unterer Teil der Hohlraumwand durch den Luftqualitätssensor gebildet wird, wobei die Schnittstelle zwischen dem Luftqualitätssensor (2) und der Skalenplatte (13) lokal abgedichtet ist, wobei die Hohlraumwand an dem seitlichen Teil eine Öffnung (130) aufweist, die durch die Membran (3) verschlossen ist.

7. Vorrichtung nach Anspruch 6, wobei die Schnittstelle zwischen dem Sensor (2) und der Skalenscheibe (13) mit mindestens einer Dichtung (81) abgedichtet ist.

8. Vorrichtung nach einem der Ansprüche 6 oder 7, wobei in der Skalenplatte (13) um die Öffnung (130) herum eine Schulter (131) von einer der Hohlraumwand gegenüberliegenden Oberfläche der Skalenplatte ausgebildet ist, wobei die Membran (3) dicht an der Schulter aufgenommen wird.

9. Vorrichtung nach Anspruch 8, bei der sich zwischen der Membran (3) und dem perforierten Bereich (140) ein Innenraum (132) befindet, der mit Hilfe einer zwischen dem Gehäuse und der Wählscheibe (13) nahe der Öffnung (130) eingefügten Dichtung (82) vom Inneren des Gehäuses (100) isoliert ist.

10. Vorrichtung nach Anspruch 1, wobei jede Durchgangsbohrung einen Durchmesser im Bereich von 0,05 bis 1,5 mm aufweist.

11. Vorrichtung nach einem der Ansprüche 1 bis 10, wobei die Vorrichtung eine elektronische Platine (16) umfasst, wobei die elektronische Platine (16) ein Loch (160) umfasst, wobei die Größe des Lochs angepasst ist, um den Luftqualitätssensor (2) durch das Loch einzuführen, wobei der Luftqualitätssensor aus beiden Seiten der elektronischen Platine austritt.

12. Vorrichtung nach Anspruch 11, die ferner eine mehrfarbige Anzeige-LED (115) umfasst, deren Farbe von der elektronischen Platine (16) in Abhängigkeit von der Konzentration eines oder mehrerer Luftschadstoffe, die von dem Luftqualitätssensor (2) erfasst werden, gesteuert werden kann.

13. Vorrichtung nach Anspruch 11, die ferner einen photoplethysmographischen (PPG) Sensor (125) umfasst, der so konfiguriert ist, dass er ein PPG-Datensignal erfasst, das die Herzfrequenz eines Benutzers anzeigt, wobei
die elektronische Platine (16) ist außerdem so konfiguriert, dass sie:
Verknüpfen des PPG-Datensignals über die Zeit mit entsprechenden Daten der Werte eines oder mehrerer Luftschadstoffe, die von dem Luftqualitätssensor erfasst werden; und
das zugehörige PPG-Datensignal und die entsprechenden Daten der von dem Luftqualitätssensor erfassten Werte eines oder mehrerer Luftschadstoffe zu speichern oder zu übertragen.

14. Vorrichtung nach Anspruch 13, die ferner eine Elektrode (124) umfasst, die so konfiguriert ist, dass sie ein elektrokardiographisches (EKG) Datensignal erfasst, das die Herzfrequenz des Benutzers anzeigt, wobei
die Elektrode eingeschaltet wird, um das EKG-Datensignal, das die Herzfrequenz des Benutzers anzeigt, auf der Grundlage einer Bestimmung zu erfassen, dass das PPG-Datensignal, das die Herzfrequenz des Benutzers anzeigt, mindestens eine Herzrhythmusstörung enthält; wobei
die elektronische Platine (16) ist außerdem so konfiguriert, dass sie:
Verknüpfen des EKG-Datensignals über die Zeit mit den entsprechenden Daten der Werte eines oder mehrerer Luftschadstoffe, die von dem Luftqualitätssensor (2) erfasst werden; und
das zugehörige EKG-Datensignal und die entsprechenden Datenpegel eines oder mehrerer vom Luftqualitätssensor erfassten Luftschadstoffe speichern oder übertragen.

## Revendications

1. Dispositif portable (9) comprenant un boîtier (100), le boîtier (100) ayant une cavité (1) avec une surface interne,
la cavité (1) contenant un capteur de qualité de l'air (2) dans la zone interne,
la cavité (1) est délimitée par une paroi de la cavité et par une membrane (3),
la membrane (3) a une paroi intérieure (30) orientée vers la zone interne de la cavité et une paroi extérieure (31) opposée à la paroi intérieure,
dans lequel
la membrane (3) est perméable à l'air ambiant et n'est pratiquement pas perméable à l'eau, ce qui permet un passage de gaz vers la zone interne de la cavité,
le boîtier (100) est formé d'une enveloppe principale (10), d'un couvercle supérieur (11) et d'une plaque arrière (12), et
l'enveloppe principale (10) comprend au moins un trou de passage (14) situé dans une zone d'ouverture (140) pour la communication de fluide entre une zone interne du boîtier et l'extérieur, la paroi externe de la membrane (31) faisant face à la zone d'ouverture (140).

2. Dispositif selon la revendication 1, dans lequel le passage du gaz vers la zone interne de la cavité se fait exclusivement à travers la membrane (3), la cavité étant par ailleurs isolée de l'intérieur du boîtier (100).

3. Dispositif selon l'une des revendications 1 ou 2, dans lequel le capteur de qualité de l'air (2) est capable de déterminer la concentration de NO₂ et/ou de CO et/ou de SO₂ et/ou de O₃.

4. Dispositif selon l'une des revendications 1 à 3, dans lequel le capteur de qualité de l'air (2) comprend un capteur ampérométrique dont la consommation d'énergie est inférieure à 5 micro-ampères et/ou dont le volume est inférieur à 0,2 cm³.

5. Dispositif selon l'une des revendications 1 à 4, dans lequel le dispositif se présente sous la forme d'un dispositif porté au poignet.

6. Dispositif selon l'une des revendications 1 à 5, dans lequel la paroi de la cavité est délimitée par une plaque de cadran (13) et par le capteur de qualité de l'air (2), la plaque de cadran (13) définissant une partie supérieure et une partie latérale de la paroi de la cavité, une partie inférieure de la paroi de la cavité étant formée par le capteur de qualité de l'air, l'interface entre le capteur de qualité de l'air (2) et la plaque de cadran (13) étant localement scellée, la paroi de la cavité comprenant sur la partie latérale une ouverture (130) obturée par la membrane (3).

7. Dispositif selon la revendication 6, dans lequel l'interface entre le capteur (2) et la plaque de cadran (13) est scellée à l'aide d'au moins un joint (81).

8. Dispositif selon l'une des revendications 6 ou 7, dans lequel un épaulement (131) est formé dans la plaque de cadran (13) autour de l'ouverture (130) à partir d'une surface de la plaque de cadran opposée à la paroi de la cavité, la membrane (3) étant reçue de manière étanche contre l'épaulement.

9. Dispositif selon la revendication 8, dans lequel un espace intérieur (132) est situé entre la membrane (3) et la zone perforée (140), l'espace intérieur (132) étant isolé de l'intérieur du boîtier (100) à l'aide d'un joint (82) inséré entre le boîtier et la plaque de cadran (13) à proximité de l'ouverture (130).

10. Dispositif selon la revendication 1, dans lequel chaque trou de passage a un diamètre compris entre 0,05 et 1,5 mm.

11. Dispositif selon l'une des revendications 1 à 10, dans lequel le dispositif comprend une carte électronique (16), la carte électronique (16) comprend un trou (160), la taille du trou étant adaptée à l'insertion du capteur de qualité de l'air (2) à travers le trou, le capteur de qualité de l'air émergeant des deux côtés de la carte électronique.

12. Dispositif selon la revendication 11, comprenant en outre un indicateur LED multicolore (115), dont la couleur est contrôlable par la carte électronique (16) en fonction d'un niveau d'un ou de plusieurs polluants atmosphériques détectés par le capteur de qualité de l'air (2).

13. Dispositif selon la revendication 11, comprenant en outre un capteur photo pléthysmographique (PPG) (125) configuré pour capturer un signal de données PPG indiquant la fréquence cardiaque de l'utilisateur ; dans lequel
la carte électronique (16) est en outre configurée pour :
associer le signal de données PPG dans le temps aux données correspondantes des niveaux d'un ou plusieurs polluants atmosphériques détectés par le capteur de qualité de l'air ; et
stocker ou transmettre le signal de données PPG associé et les données correspondantes des niveaux d'un ou plusieurs polluants atmosphériques détectés par le capteur de qualité de l'air.

14. Dispositif selon la revendication 13, comprenant en outre une électrode (124) configurée pour capturer un signal de données électrocardiographiques (ECG) indiquant la fréquence cardiaque de l'utilisateur ; dans lequel
l'électrode est mise sous tension afin de capturer le signal de données ECG indiquant la fréquence cardiaque de l'utilisateur sur la base d'une détermination que le signal de données PPG indiquant la fréquence cardiaque de l'utilisateur comprend au moins une arythmie cardiaque ; dans lequel
la carte électronique (16) est en outre configurée pour :
associer le signal de données ECG dans le temps aux données correspondantes des niveaux d'un ou plusieurs polluants atmosphériques détectés par le capteur de qualité de l'air (2) ; et
stocker ou transmettre le signal de données ECG associé et les niveaux de données correspondants d'un ou plusieurs polluants atmosphériques détectés par le capteur de qualité de l'air.
